# FASCICULE DE BREVET EUROPEEN

(11) **EP 2 992 082 B1**
(45) Date de publication et mention de la délivrance du brevet: **07.06.2017**
(21) Numéro de dépôt: 14727599.4
(22) Date de dépôt: 29.04.2014
(51) Int. Cl.: C12M 1/22, C12M 1/00, C12M 1/26

(54) **DISPOSITIF ET PROCEDE POUR LA REPARTITION D'UNE SUSPENSION DE MICROORGANISMES**
VORRICHTUNG UND VERFAHREN ZUR AUSGABE EINER SUSPENSION AUS MIKROORGANISMEN
DEVICE AND METHOD FOR DISPENSING A SUSPENSION OF MICROORGANISMS

(30) Priorité: 03.05.2013 FR 1354091
(43) Date de publication de la demande: 09.03.2016
(73) Titulaire: Biomérieux, 69280 Marcy l'Étoile (FR)
(72) Inventeur: GORSE, Florence, F-69380 Charnay (FR); COLIN, Bruno, F-69280 Marcy l'Etoile (FR)
(74) Mandataire: Sprugnoli, Claude Louis Victor
(86) Numéro de dépôt international: PCT/FR2014/051028
(87) Numéro de publication internationale: WO 2014/177807

(56) Documents cités:
- FR-A1- 2 639 957
- FR-A1- 2 686 895
- US-A- 2 348 448
- US-A- 3 907 647
- US-A- 4 321 330

## Description

Le domaine technique de la présente invention est celui du diagnostic in vitro et plus précisément, celui du diagnostic microbiologique. La présente invention concerne en particulier un dispositif comprenant un applicateur apte à répartir une suspension de microorganismes sur un milieu de culture. L'invention concerne également un procédé d'isolement de microorganismes dans une suspension de microorganismes ainsi qu'un procédé de dénombrement de microorganismes dans une suspension de microorganismes.

L'utilisation des milieux de culture dans le domaine microbiologique est connue depuis de nombreuses décennies pour permettre la croissance, la détection, l'isolement et le dénombrement des microorganismes. Ces milieux de culture se retrouvent communément sous forme de gélose contenue dans une boîte de Petri ou sous forme déshydratée appliquée sur un support, généralement un film.

Les milieux de culture déshydratés sur support ont été développés pour remplacer la boite de Petri. Pour ces produits, la réhydratation se fait in situ, c'est à dire directement dans l'espace servant à l'inoculation et à l'incubation. L'un d'eux, le système Petrifïlm^{™}, comprenant des nutriments réhydratables, est très largement utilisé. Un autre système développé par la société Nissui Pharmaceutical, le Compact Dry, se présente également sous forme de milieux déshydratés. Ces milieux de culture ont pour avantage de se conserver plus longtemps qu'un milieu de culture gélosé prêt à l'emploi. Ils peuvent également, comme Petrifilm^{™}, présenter un faible encombrement et ainsi utiliser un faible espace d'incubation. La surface disponible pour la culture est limitée et les nutriments disponibles par cellule sont de concentration adaptée pour donner des colonies de diamètre plus petit que sur milieu gélosé classique, la forme des colonies pouvant être aussi modifiée du fait de la faible solidité du gel utilisé ou des coefficients élevés de diffusion des bactéries.

L'isolement de microorganismes sur milieu de culture, à partir d'un échantillon à analyser ou d'une suspension de microorganismes, est une étape souvent indispensable à de nombreux procédés d'analyse microbiologique. Cette étape est notamment utilisée pour réaliser des identifications, vérifier la pureté microbienne d'un échantillon ou encore effectuer un dénombrement bactérien par comptage des colonies isolées ainsi obtenues. Après l'incubation, on dénombre les colonies en milieu gélosé déposé en boite de Petri ou sur autres supports. Le dénombrement peut se faire à l'oeil ou grâce à un dispositif automatisé de dénombrement de colonies. Connaissant la quantité de l'inoculum de l'échantillon et les rapports de dilution, on peut déterminer le nombre de microorganismes par unité de volume ou par unité de poids de l'échantillon, habituellement exprimé en UFC/ml ou en UFC/g (Unité Formant Colonie), à partir du nombre de colonies dénombrables.

Les techniques d'isolement ont pour objectif d'obtenir à la surface d'un milieu nutritif gélifié des colonies directement utilisables (CDU). Elles sont bien connues de l'homme du métier, la technique des stries étant la technique de référence. Cette dernière consiste à déposer l'inoculum par frottement sur une surface avec une probabilité égale par unité de surface parcourue. La densité locale distribuée diminue approximativement de façon exponentielle lors du parcours de l'instrument. Ainsi, plusieurs zones d'étalement à partir d'un seul inoculum sont réalisées, avec ou sans chevauchements des zones, afin d'obtenir l'effet adéquat de distribution et un appauvrissement en bactéries lors du segment d'étalement subséquent. En fin d'étalement les cellules sont suffisamment isolées les unes des autres pour que les développements microbiens sous forme de CDU (colonies visibles ou microcolonies) ne soient pas superposés, même partiellement. Une autre technique largement répandue, notamment pour dénombrer des microorganismes, consiste en l'isolement sur boîte de milieu gélifié par étalement en surface. Dans ce cas, un mélange de cellules à une concentration cellulaire faible permettant de mettre en culture jusqu'à 300 cellules est étalé à la surface du gel d'une boîte de Petri de 9 cm de diamètre, chaque cellule se développant en une colonie isolée. Cependant, lorsque moins de 5 cellules sont mises en contact avec le gel nutritif des problèmes statistiques faussent l'exactitude du dénombrement. D'autre part, lorsque l'effectif dénombré est au-delà de 150 ou 300 cellules, des erreurs de dénombrement peuvent apparaître du fait du chevauchement des surfaces des colonies, ou d'éventuelles interactions. Dans d'autres cas de concentrations supérieures à 300 UFC/ml (Unité Formant Colonie ou Colony Forming Unit - cfu), une culture bactérienne aura tendance à former seulement des micro-colonies du fait du manque de milieu nutritif. Ces micro-colonies n'étant pas toujours visibles à l'oeil nu, le résultat de cette culture peut être analysé comme faussement négatif. L'étalement est habituellement réalisé par un instrument comportant par exemple, une partie linéaire qui est en contact avec le gel ou par emploi de billes de quelques millimètres de diamètre roulant aléatoirement sur la surface par un mouvement désordonné. Cette technique n'est donc adaptée qu'à un échantillon faiblement contaminé ou dilué car un nombre élevé de cellules augmente la probabilité de confluence des colonies résultant de la croissance.

Il est également possible d'effectuer un dénombrement sur boite par ensemencement dans la masse de la gélose. L'échantillon de départ est dilué de manière à réduire suffisamment la population microbienne et obtenir des colonies séparées. De petits volumes connus de chacun des échantillons à dénombrer et éventuellement dilués sont alors mélangés à un gel liquide, habituellement de la gélose maintenue en surfusion environ à 45°C. Les mélanges sont réalisés dans des boites de culture stériles et après gélification et incubation, chaque cellule est immobilisée et forme une colonie par multiplication cellulaire.

Dans le cas des milieux de culture déshydratés sur support, l'isolement de colonies n'est possible que par inclusion dans le gel formé lors de la réhydratation, et donc à partir d'un échantillon de départ faiblement contaminé ou ayant subi une série de dilutions. La concentration finale, à déposer sur le milieu, doit être inférieure à 250 ou 100 UFC/ml (Unité Formant Colonie ou Colony Forming Unit - cfu), ces données classiques dépendant du milieu utilisé. L'inoculum est versé sur le milieu déshydraté et peut être étalé sur la surface grâce à un applicateur. Cet applicateur permet notamment de répartir de façon uniforme l'inoculum. Un applicateur connu est le Petrifïlm^{™} spreader de la société 3M^{™}.

Néanmoins, lorsque l'échantillon de départ est largement contaminé (au-delà de 300 Unités Formant Colonie/ml), l'utilisation de ces techniques nécessite la réalisation d'une série de dilutions, impliquant une prise d'essais plus importante, une perte de temps et la consommation d'un nombre important de réactifs (milieu de culture, tubes de diluants, etc.), générateur d'un volume élevé de déchets (autoclavage, coût du traitement). De plus, si un grand nombre de dilutions est réalisé, il existe un risque de perdre, par l'effet de la dilution, le microorganisme cible, si celui-ci est présent en faible quantité par rapport à la microflore totale.

Le document US 3632478 décrit un applicateur (20) pour milieux de culture gélosé présentant une surface convexe afin de chasser d'éventuelles bulles présentes dans la suspension, ainsi qu'une dent (24) destinée à pénétrer le milieu de culture afin de l'ensemencer en profondeur. Cet applicateur est destiné à rester en position de contact avec le milieu de culture afin de favoriser une croissance en anaérobie des éventuels microorganismes présents dans la suspension. Cependant, cet applicateur ne peut être utilisé sur des milieux déshydratés ou sur des milieux gélosés de toute épaisseurs. D'autre part, la forme convexe de la surface de contact ne permet pas de répartir la suspension selon des épaisseurs différentes. Enfin l'applicateur étant destiné à rester en contact avec le milieu de culture, une lecture du nombre d'Unité Formant Colonies après incubation du milieu peut être faussée par le matériau employé. Dans le cas où l'applicateur serait retiré, certaines colonies pourraient également adhérer à la surface de contact et venir contaminer d'autre surfaces.

Le document EP-B1-1363991 propose un milieu de culture déshydraté présentant un film flexible destiné à répartir de manière homogène une suspension déposée sur un milieu de culture déshydraté. Pour cela, une surépaisseur présentant une ouverture circulaire est déposé sur la surface du substrat constituant le milieu de culture. La suspension est déposée sur le substrat, au centre de l'ouverture circulaire formée par la surépaisseur, le film flexible est alors rabattu sur la suspension afin de la répartir uniformément jusqu'à atteindre les bords formés par la surépaisseur. Une fois répartie, la suspension présente donc une surface d'épaisseur uniforme contenue par l'ouverture circulaire de la surépaisseur. Ce dispositif ne permet cependant pas de répartir la suspension de manière non uniforme sur le milieu. D'autre part, une quantité connue de suspension doit être déposée sur le milieu afin de limiter les risques d'écoulement.

Le document FR-A1-2686895 décrit une boîte de culture destinée à la culture des microorganismes comprenant un réceptacle qui est coiffé d'un couvercle et sur le fond duquel est disposée une couche d'un milieu de culture. Le dispositif comprend également un étaleur issu d'une face intérieure du couvercle et destiné à étaler un inoculum sur la couche nutritive. L'étaleur ou applicateur ne permet cependant pas de répartir la suspension selon des épaisseurs différentes. L'utilisation avec un milieu de culture déshydraté n'est également pas décrite.

Le document FR-A1-2594848 propose de mélanger un milieu avec un échantillon et de le solidifier dans une boîte de Petri comportant différent compartiments de hauteurs définies. Le milieu ainsi réparti forme des couches de gélose d'épaisseurs différentes. La géométrie connue de la boîte permet ainsi de calculer la quantité de microorganismes dans l'échantillon principal, à partir du nombre de colonies comptées dans un compartiment de volume définit. Ce document permet de s'affranchir des dilutions successives mais n'est pas applicable à un milieu de culture déshydraté, notamment sous forme de film. L'utilisation de ce milieu nécessite d'autre part de maintenir le milieu de culture à une température suffisante pour le mélanger à l'échantillon puis d'attendre la solidification complète du milieu avant incubation puis dénombrement.

Il ressort de l'état de la technique considéré qu'il n'existe pas de dispositif ou de procédé d'isolement et de dénombrement, simple à mettre en oeuvre, à partir d'un échantillon à analyser ou d'une suspension de microorganismes de concentration inconnue, et qui permet d'obtenir des colonies isolées ou des colonies dénombrables sur milieu gélosé ou déshydraté sur support.

Un premier objectif de la présente invention est de fournir un dispositif comprenant un applicateur apte à répartir une suspension de microorganismes sur un milieu de culture et le processus d'isolement associé, plus performant que les méthodes et dispositifs de l'état de la technique.

Un deuxième objectif de la présente invention est de fournir un dispositif comprenant un applicateur apte à répartir une suspension de microorganismes sur un milieu de culture en au moins deux volumes de suspension d'épaisseurs distinctes.

Un troisième objectif de la présente invention est de fournir un procédé d'isolement de microorganismes à partir d'un échantillon ayant une concentration en microorganismes initiale élevée.

Un quatrième objectif de la présente invention est de fournir un procédé d'isolement compatible également avec le dénombrement des microorganismes.

Un cinquième objectif de la présente invention est de fournir un applicateur et un procédé d'isolement compatible avec l'utilisation de milieux déshydratés sur support.

Un sixième objectif de la présente invention est de fournir un dispositif et un procédé correspondant permettant de réaliser un dénombrement de manière plus simple et moins couteuse que les méthodes et dispositifs actuels.
Ces objectifs parmi d'autres sont atteints par la présente invention qui propose un dispositif comprenant un applicateur apte à répartir une suspension de microorganismes sur un milieu de culture lui-même déposé sur un support. Dans sa position de contact avec la suspension de microorganismes, l'applicateur comprend au moins deux surfaces de contact avec la suspension de microorganismes, chaque surface définit un plan, les au moins deux plans étant parallèles l'un par rapport à l'autre et étant distants d'une hauteur h, les au moins deux plans parallèles font face à l'interface entre ladite suspension de microorganismes et le milieu de culture et sont également disposés parallèlement à ladite interface.
On entend par applicateur un dispositif apte à répartir une suspension de microorganismes sur un milieu de culture. Cette répartition peut être sous la forme d'une couche de suspension d'épaisseur uniforme ou de plusieurs épaisseurs. Un applicateur selon l'invention peut être réalisé avec une grande variété de matériaux connus de l'homme du métier tel que des plastiques, métaux, cartons. Des plastiques pouvant être utilisés sont, par exemple et de manière non limitative, le polystyrène, le polyéthylène ou le polypropylène. L'utilisation de plastiques peu couteux permet de rendre le dispositif à usage unique et jetable afin de limiter les risques de contamination. Le matériau employé peut être rigide ou flexible pour permettre de répartir la suspension. Le matériau ne doit pas être poreux pour ne pas absorber la suspension lors de la répartition. Le matériau peut ainsi être hydrophobe.
La répartition peut se faire directement par contact de l'applicateur sur la suspension de microorganismes ou alternativement en plaçant l'applicateur sur un film protecteur disposé sur la suspension. L'applicateur peut être disposé manuellement ou de manière automatique. La force nécessaire pour répartir la suspension peut être obtenue par la masse de l'applicateur, la force appliquée par l'utilisateur ou tout autre dispositif semi-automatisé ou automatisé connu de l'homme du métier.
Au sens de la présente invention, une surface de contact est une surface de l'applicateur apte à rentrer en contact avec la suspension de microorganismes afin de la répartir, éventuellement par l'intermédiaire d'un film protecteur. Une surface de contact est une surface sensiblement plane. Le contour du plan formé par la surface de contact peut prendre une grande variété de formes. Il peut notamment être circulaire, de sorte qu'au moins deux surfaces de contact forment deux cercles concentriques. Le contour du plan formé par la surface de contact peut également être une portion quelconque d'un cercle, par exemple formé par deux rayons partant du centre et un arc de cercle. Il peut également être rectangulaire.
Les aux moins deux surfaces de contact de l'applicateur permettent de répartir la suspension en au moins deux volumes de suspension d'épaisseurs distinctes. Les aux moins deux surfaces de contact définissent au moins deux plans parallèles. La hauteur h correspond à la plus petite distance entre les au moins deux plans parallèles. La hauteur h permet de définir la différence d'épaisseur obtenue après répartition de la suspension entre deux surfaces de contact. Dans le cas d'un applicateur comportant seulement deux surfaces de contact la hauteur h permet de définir la différence d'épaisseur des deux volumes de suspension répartis par l'applicateur. Les aux moins deux surfaces de contact peuvent être juxtaposées ou imbriquées. Dans le cas où les deux surfaces sont imbriquées, au moins une partie de la première surface de contact forme alors saillie dans le plan formé par la seconde surface de contact. Alternativement au moins une partie de la première surface de contact peut former un évidemment dans le plan formé par la seconde surface de contact. Alternativement, la zone située entre les deux surfaces de contact, qu'elles soient juxtaposées ou imbriquées, est matérialisée par un moyen de marquage. Ce moyen de marquage permet de matérialiser la frontière entre les deux surfaces de contact lorsque celles-ci sont disposées sur le milieu de culture. Ce moyen de marquage permet ainsi d'améliorer le dénombrement des colonies présentes sur la partie supérieure du milieu en séparant les zones de concentrations différentes. Ce moyen de marquage peut être réalisé par exemple par un ajout de matière sur la surface de contact de l'applicateur, continu ou discontinu permettant d'embosser la partie supérieure du milieu de culture. Alternativement ce moyen de marquage peut être réalisé par un évidemment, un tampon encreur, une lame , un alignement de poinçons ou tout autre moyens, disposés dans la zone située entre les deux surfaces de contact. Alternativement, ce moyen de marquage matérialise uniquement la frontière entre deux surfaces de contact de manière visuelle et ne modifie pas l'épaisseur de suspension répartie ou l'épaisseur du milieu de culture.
On entend par milieu de culture, un milieu comprenant tous les constituants nécessaires à la survie et/ou à la croissance de microorganismes, déposé sur un support. En pratique, l'homme du métier choisira le milieu de culture en fonction des microorganismes cibles, selon des critères parfaitement connus et à la portée de cet homme de l'art. Un milieu de culture peut se présenter sous une forme déshydratée ou gélosée. Dans le cas de la forme gélosée, le milieu de culture est contenu dans une boîte de Petri. Le support du milieu de culture est dans ce cas le fond plan de la boîte de Petri. Les constituants sont mélangés à chaud et déposés sous forme liquide dans la boîte. En refroidissant le mélange formé se solidifie, la suspension peut alors être déposée sur la partie supérieure du milieu.
Dans le cas de la forme déshydratée ou séchée ou lyophilisée, les constituants ou une partie des constituants nécessaires à la survie et/ou à la croissance de microorganismes sont appliqués sur un ou plusieurs supports solides sous forme sèche. La suspension est alors déposée sur ou entre ces supports solides. Ces supports, parfois flexibles, sont maintenus plans lors de l'utilisation du milieu. Au contact de la suspension, les constituants sous forme sèche se réhydratent et assurent leur fonction. Cette forme de milieu est particulièrement avantageuse pour conserver les constituants ainsi que pour limiter l'encombrement du milieu.
On entend par suspension de microorganismes un échantillon liquide ou visqueux susceptible de contenir un ou plusieurs microorganismes. Au sens de la présente invention, le terme microorganisme recouvre les bactéries à Gram positif ou à Gram négatif, les levures, les moisissures, les amibes et plus généralement, les organismes unicellulaires, invisibles à l'oeil nu, qui peuvent être manipulés et multipliés en laboratoire.
Selon un mode préféré de réalisation de l'invention, le microorganisme est une bactérie, à Gram négatif ou positif, une levure, ou une moisissure.
A titre d'exemple de bactéries à Gram positif, on peut citer les bactéries des genres suivants : *Enterococcus, Streptococcus, Lactobacillus, Bifidobacterium, Staphylococcus, Bacillus, Listeria, Clostridium, Mycobacteria, Nocardia, Corynebacteria, Micrococcus* et *Deinococcus.*
A titre d'exemple de bactéries à Gram négatif, on peut citer les bactéries des genres suivants :
*Escherichia, Enterobacter, Klebsiella, Salmonella, Proteus, Serratia.*
A titre d'exemple de levures, on peut citer les genres suivants : *Candida, Cryptococcus, Saccharomyces et Trichosporon.*
A titre d'exemple de moisissures, on peut citer les genres suivants : *Aspergilles, Penicillium, Cladosporium.*

On entend par interface la zone de contact entre la partie supérieure du milieu de culture et la suspension de microorganismes une fois déposée sur le milieu de culture. L'interface forme généralement un plan. Dans le cas d'un milieu gélosé, le milieu est déposé à chaud sur le support et se solidifie en refroidissant pour former une partie supérieure plane. La suspension de microorganismes étant liquide, l'interface entre la suspension déposée et la partie supérieure du milieu de culture se forme alors sur le même plan. Dans le cas d'un milieu déshydraté, au moins une partie des constituants du milieu de culture est déposée sur un support plan puis déshydratée, ou directement sous forme déshydratée. Dans ces deux cas, la partie supérieure d'au moins une partie des constituants du milieu de culture forme un plan.

On entend par partie supérieure du milieu de culture la partie sur laquelle la suspension de microorganismes est déposée lors de l'inoculation du milieu.

La présente invention concerne également un dispositif comprenant un applicateur apte à répartir une suspension de microorganismes sur un milieu de culture. Dans sa position de contact avec la suspension de microorganismes, l'applicateur comprend au moins deux surfaces de contact avec la suspension de microorganismes, chaque surface définit un plan, les au moins deux plans étant parallèles l'un par rapport à l'autre et étant distants d'une hauteur h, les au moins deux plans parallèles font face à l'interface entre ladite suspension de microorganismes et le milieu de culture et sont également disposés parallèlement à ladite interface, h étant égal à h₁-h₂, h₁ étant la distance entre la première surface de contact et l'interface, h₂ étant la distance entre la seconde surface de contact et l'interface et le ratio h₁/h₂ étant supérieur à 1 et inférieur ou égal à 100.
De préférence, le ratio h₁/h₂ est compris entre 2 et 10. Plus préférentiellement ce ratio est égal à 3.

S1 correspond à l'aire de la première surface de contact de l'applicateur et S2 correspond à l'aire de la seconde surface de contact de l'applicateur.

Dans un mode de réalisation particulier, le ratio (h₁*S₁)/(h₂*S₂) est supérieur à 1 et inférieur ou égal à 1000.

Dans un mode de réalisation particulier, en vue perpendiculaire aux plans parallèles formés par les surfaces de contacts, les aires S₁ et S₂ ne se chevauchent pas, de même que les surfaces de contact.

La valeur du ratio h1/h2 permet de définir un rapport d'épaisseur de la suspension répartie par les surfaces de contact des zones de l'applicateur. Les volumes respectifs de suspension répartis par ces deux surfaces sont h₁*S₁ et h₂*S₂. Ainsi, si les aires des surfaces S₁ et S₂ sont égales, la valeur du ratio h1/h2 permet de définir le ratio entre les deux volumes de suspension répartis par les deux surfaces de contact de l'applicateur.

De façon plus générale, d'autre ratios hₙ/hₙ₊₁, pourront être aisément définis par l'homme du métier. hₙ étant la distance entre la nième surface de contact et l'interface et Sₙ correspond à l'aire de la nième surface de contact. De même, hₙ*Sₙ déterminera le volume réparti par la nième surface de contact.

L'intérêt de l'invention est donc de fournir un dispositif comprenant un applicateur apte à répartir un volume de suspension en au moins deux volumes d'épaisseurs et/ou de surfaces différentes. L'aire occupée par la surface d'un volume de suspension répartie étant mesurée dans le plan parallèle à la surface de contact de l'applicateur. Dans un mode de réalisation particulier, l'applicateur répartit la suspension en au moins deux volumes de surfaces égales et d'épaisseurs différentes. Dans un autre mode de réalisation, l'applicateur répartit la suspension en au moins deux volumes de surfaces différentes et d'épaisseurs différentes.

Ainsi, une dilution artificielle est réalisée à partir du volume initial de suspension, celui-ci étant réparti sur au moins deux volumes de surfaces et d'épaisseurs connues. Le rapport entre les deux volumes et alors équivalent à un rapport de dilution. Suivant le microorganisme recherché dans la suspension, la hauteur h et les aires des au moins deux surfaces seront adaptées pour permettre les dilutions souhaitées entre les différents volumes et surfaces correspondant. Par exemple, la suspension peut être artificiellement diluée au tiers par une zone où l'épaisseur sera trois fois moins importante par rapport à une autre zone dont la surface est identique. Le dénombrement sera donc plus aisé dans cette zone où la suspension est artificiellement moins concentrée, notamment dans le cas où la suspension est très riche en microorganismes. Des rapports de dilution utilisés sont par exemple et de manière non limitative : 1/3 ou 10⁻¹ pour une suspension de microorganisme peu concentrée, de 10⁻², 10⁻³, 10⁻⁴, 10⁻⁵ ou 10⁻⁶. pour une flore totale.

Dans un mode de réalisation particulier, le dispositif comprend au moins un moyen d'espacement entre les surfaces de contact de l'applicateur et le support ou l'interface. Alternativement le moyen d'espacement est solidaire de l'applicateur. Alternativement le moyen d'espacement est indépendant de l'applicateur tel qu' au moins une entretoise rapportée.

Ce moyen d'espacement assure une distance minimale entre au moins une surface de contact de l'applicateur et la partie supérieure du milieu de culture pour garantir une épaisseur de suspension minimale quelle que soit la force appliquée sur l'applicateur lors de la répartition. Alternativement le moyen d'espacement est indépendant de l'applicateur tel qu'une entretoise rapportée, indépendante de l'applicateur et pouvant être retirée après la répartition de la suspension sans avoir à retirer l'applicateur.

Il n'est pas nécessaire que les aires des aux moins deux surfaces de contact, S1 et S2, ne recouvrent la totalité de la surface du milieu de culture. Les aires des aux moins deux surfaces de contact peuvent notamment recouvrir une partie seulement de l'aire de la partie supérieure du milieu de culture. A cet effet, le dispositif comprend un moyen d'espacement entre les surfaces de contact de l'applicateur et le support ou l'interface assurant une distance minimale entre au moins une surface de contact de l'applicateur et la partie supérieure du milieu de culture. Un volume de suspension de microorganismes plus important que la somme des volumes à répartir, par exemple (h₁*S₁) et (h₂*S₂), peut ainsi être déposé. De cette manière, même si un excès de suspension de microorganismes s'écoule hors des aires des surfaces de contact S₁ et S₂, hors de l'interface, ou hors du dispositif, ne pouvant ainsi plus être répartis, les volumes répartis par le dispositif selon l'invention restent corrects. De façon préférentielle, un agent gélifiant pouvant être compris dans un milieu de culture déshydraté, sur les surfaces de contact de l'applicateur, dans la suspension de microorganisme ou sur le film protecteur disposé sur la suspension, permet de ralentir l'écoulement de la suspension et de la solidifier rapidement (en moins d'une minute, idéalement en quelques secondes) lorsque celle-ci est déposée sur le milieu de culture. De cette manière, la suspension peut être répartie en plusieurs volumes à répartir avant qu'il ne reste, au contact du dispositif selon l'invention, un volume de suspension inférieur à la somme des volumes à répartir ; signifiant qu'un volume de suspension trop important s'est écoulé hors des aires des surfaces de contact S₁ et S₂, de l'interface, ou hors du dispositif, ce volume écoulé ne pouvant plus être répartis.

Alternativement la distance minimale entre au moins une surface de contact de l'applicateur et la partie supérieure du milieu de culture peut être garantie par des moyens robotisés connus de l'homme du métier. Alternativement cette distance minimale est garantie par un dispositif, manuel ou automatisé assurant une force définie d'application de l'applicateur sur la suspension.

Dans un mode de réalisation particulier, le dispositif comprend un moyen périphérique de confinement de la suspension de microorganismes. Ce moyen périphérique de confinement permet de retenir la suspension déposée sur le milieu afin qu'elle ne déborde pas du milieu de culture lors de la répartition par l'applicateur. Des moyens périphériques de confinement peuvent être réalisés par une surépaisseur de matière sur l'applicateur et / ou sur le milieu de culture et / ou son support. Alternativement, ces moyens périphériques de confinement peuvent être réalisés par un film déposé autour du milieu de culture comprenant au moins une ouverture de forme quelconque. Cette surépaisseur peut éventuellement former une bague, une surépaisseur discontinue ou toute forme susceptible de confiner la suspension connue de l'homme du métier. Avantageusement les moyens périphériques de confinement peuvent assurer la fonction du moyen d'espacement afin d'assurer une distance minimale entre au moins une surface de contact de l'applicateur et la partie supérieure du milieu de culture. Avantageusement, la surépaisseur est discontinue afin d'évacuer de manière contrôlée une quantité trop importante de suspension déposée sur la partie supérieure du milieu de culture.

Avantageusement, le moyen périphérique de confinement permet de confiner un volume de suspension supérieur à la somme des volumes répartis par le dispositif selon l'invention, de manière à confiner un excès de volume de suspension ayant été déposé sur le milieu de culture.

L'invention concerne également un procédé d'isolement de microorganismes contenus dans une suspension de microorganismes utilisant un dispositif selon l'invention comprenant les étapes suivantes :
a) déposer une suspension sur un milieu de culture lui-même déposé sur un support ;
b) placer un applicateur sur la suspension, ledit applicateur répartissant la suspension en au moins deux volumes différents et déterminés,
c) incuber le milieu de culture pendant un temps et à une température prédéterminée permettant la croissance des microorganismes ;

L'invention concerne également un procédé d'isolement de microorganismes contenus dans une suspension de microorganismes utilisant un dispositif selon l'invention comprenant les étapes suivantes :
a) déposer une suspension sur un milieu de culture lui-même déposé sur un support
a') placer un film protecteur sur la suspension,
b) placer un applicateur sur le film, ledit applicateur répartissant la suspension en au moins deux volumes différents et déterminés,
c) incuber le milieu de culture pendant un temps et à une température prédéterminée permettant la croissance des microorganismes ;

Dans le cas d'une utilisation du dispositif selon l'invention pour la répartition d'une suspension de microorganismes sur un milieu de culture, il n'est pas nécessaire d'utiliser un moyen périphérique de confinement, notamment grâce à l'action d'un agent gélifiant, celui-ci pouvant être compris : dans un milieu de culture déshydraté, sur les surfaces de contact de l'applicateur, dans la suspension de microorganisme ou sur le film protecteur disposé sur la suspension.

Ainsi, selon un procédé particulier d'utilisation du dispositif selon l'invention, un procédé d'isolement de microorganismes contenus dans une suspension peut comprendre les étapes suivantes :
a) déposer une suspension sur un milieu de culture lui-même déposé sur un support ;
a") attendre la gélification de la suspension de microorganisme durant moins d'une minute, de manière à pouvoir la répartir sans que celle-ci ne s'écoule de manière trop importante hors de l'interface, hors des aires des surfaces de contact S₁ et S₂ ou hors du dispositif,
b) placer un applicateur sur la suspension, ledit applicateur répartissant la suspension en au moins deux volumes différents et déterminés,
c) incuber le milieu de culture pendant un temps et à une température prédéterminée permettant la croissance des microorganismes ;
Alternativement, une étape consistant à :
a') placer un film protecteur sur la suspension,
pourra être réalisée entre l'étape a) et b) du procédé précédemment décrit.
L'invention concerne également un procédé d'isolement de microorganismes contenus dans une suspension de microorganismes utilisant un dispositif selon l'invention comprenant une étape supplémentaire d) consistant à dénombrer les colonies issues de ces microorganismes.

On entend par film protecteur un film amovible pouvant être disposé sur la suspension de microorganismes une fois celle-ci déposée sur le milieu de culture. Les films protecteurs peuvent être réalisés, par exemple et de manière non limitative, en polyéthylène, polypropylène ou polychlorure de vinyle. L'épaisseur de film est préférentiellement comprise entre 30 et 120µm. Dans le cas où un film protecteur est disposé entre la suspension et la surface de contact de l'applicateur, l'applicateur peut être réutilisé sans procéder à un lavage. Alternativement, le film protecteur est intégré à l'applicateur. Alternativement, l'applicateur selon l'invention est solidaire du support du milieu de culture.

Alternativement, un agent gélifiant peut être déposé sur la face interne du film protecteur. La face interne du film protecteur est la face qui entre en contact avec la suspension de microorganismes. Un agent gélifiant est un agent se solidifiant à température ambiante au contact d'un liquide, notamment d'une suspension de microorganismes. De nombreux agents gélifiants peuvent être utilisés tel que et de manière non limitative : gomme de guar, gomme de xanthane, gomme de caroube, hydroxyéthylcellulose, carboxyméthylcellulose, polyacrylamide, algine, caragean, cire, silicone et des combinaisons de ces agents. Un agent gélifiant permet de solidifier une suspension de microorganismes déposée sur un milieu de culture en moins d'une minute, généralement en quelques secondes, de sorte que la suspension peut être répartie par le dispositif selon l'invention sans qu'un volume trop important ne s'écoule hors de l'interface, des aires des surfaces de contact, ou de la zone de croissance du milieu de culture, idéalement sans que la suspension ne s'écoule ou ne fuite hors de l'interface, des aires des surfaces de contact, ou de la zone de croissance du milieu de culture. Un volume écoulé trop important signifie que le volume de suspension pouvant encore être répartis par le dispositif selon l'invention après que celle-ci ait été déposée et ce soit écoulée est inférieur à la somme des volumes à répartir définis par le dispositif selon l'invention.

Alternativement, un agent gélifiant peut être ajouté à la suspension. Cet ajout permet notamment d'améliorer l'utilisation d'un dispositif selon la présente invention avec un milieu gélosé. Dans ce mode de réalisation, la suspension contenant l'agent gélifiant est répartie sur un milieu gélosé avec l'applicateur selon l'invention. Après solidification de l'agent gélifiant, et donc de la suspension, l'applicateur peut ainsi être retiré notamment pour faciliter la lecture de la pousse après incubation.

Alternativement, l'invention concerne également un procédé de dénombrement de microorganismes contenus dans une suspension de microorganismes utilisant un dispositif selon l'invention comprenant les étapes suivantes :
a) déposer une suspension sur un milieu de culture lui-même déposé sur un support;
b) placer un applicateur sur la suspension, ledit applicateur répartissant la suspension en au moins deux volumes différents,
c) incuber le milieu de culture pendant un temps et à une température prédéterminée permettant la croissance des microorganismes ;
d) dénombrer les colonies issues de ces microorganismes.

Alternativement, l'invention concerne également un procédé de dénombrement de microorganismes contenus dans une suspension de microorganismes utilisant un dispositif selon l'invention comprenant les étapes suivantes :
a) déposer une suspension sur un milieu de culture lui-même déposé sur un support;
a') placer un film protecteur sur la suspension
b) placer un applicateur sur le film, ledit applicateur répartissant la suspension en au moins deux volumes différents et déterminés,
c) incuber le milieu de culture pendant un temps et à une température prédéterminée permettant la croissance des microorganismes ;
d) dénombrer les colonies issues de ces microorganismes.
Alternativement, le milieu de culture est déshydraté, séché ou lyophilisé.
Alternativement, le procédé d'isolement ou de dénombrement selon l'invention comprend une étape préliminaire à l'étape b) consistant à placer une entretoise rapportée sur le milieu de culture.
Alternativement, le procédé d'isolement ou de dénombrement selon l'invention comprend une étape préliminaire à l'étape a) consistant à placer un moyen périphérique de confinement sur le milieu de culture.

L'invention concerne également l'utilisation d'un dispositif selon l'invention pour la mise en oeuvre d'un procédé d'isolement ou de dénombrement de microorganismes.

L'invention sera mieux comprise à la lecture des exemples nullement limitatifs qui suivent, en référence aux dessins. Par simplification, les parties ou éléments d'une forme de réalisation qui se retrouvent de manière identique ou similaire dans une autre forme de réalisation seront identifiés par les mêmes références numériques et ne seront pas à nouveau décrits.

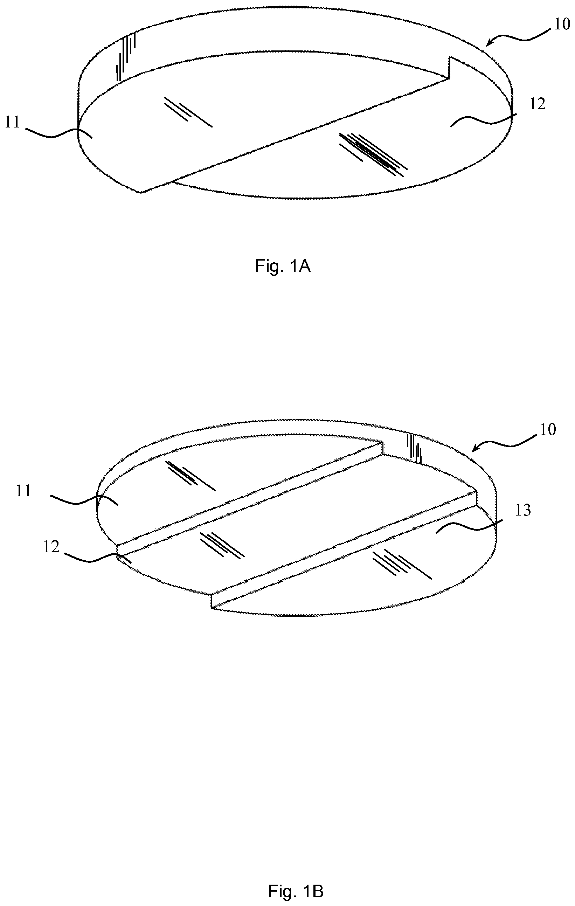

Les figures 1A et 1B sont des représentations schématiques de l'applicateur compris dans le dispositif selon l'invention.

L'applicateur (10) comprend au moins deux surfaces de contact (11,12) avec la suspension de microorganismes sur au moins deux plans parallèles distants d'une hauteur h.
La figure 1A représente un mode de réalisation particulier de l'invention comprenant une première surfaces de contact (11) ainsi qu'une deuxième surface de contact (12).
La figure 1B représente un mode alternatif de réalisation de l'invention comprenant une première surface de contact (11), une deuxième surface de contact (12) ainsi qu'une troisième surface de contact (13).
   La forme de l'applicateur (10) telle que représentée dans les figures n'est pas limitative, celui-ci pouvant être cylindrique, parallélépipédique, en tronc de cône ou toute formes connues de l'homme du métier susceptibles de présenter au moins deux surfaces de contact avec la suspension de microorganismes sur au moins deux plans parallèles distants d'une hauteur h. L'axe principal de la forme de l'applicateur étant généralement perpendiculaire au plan formé par une surface de contact. Le dispositif peut alternativement présenter un moyen de préhension situé sur la surface opposée aux surfaces de contact. Ce moyen de préhension facilite la manipulation de l'applicateur par un opérateur, notamment lors de la répartition de la suspension.

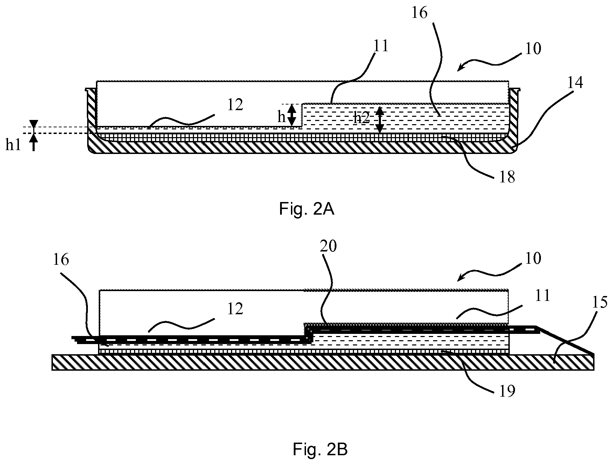

Les figures 2A et 2B sont des représentations schématiques du dispositif selon l'invention.
La figure 2A représente l'applicateur (10) ayant réparti une suspension (16) sur un milieu de culture (18) disposé dans une boite de Petri (14). Les deux surfaces de contact (11, 12) avec la suspension de microorganimes (16) font face à l'interface entre la suspension de microorganisme et le milieu de culture et répartissent la suspension sur deux épaisseurs. Les deux surfaces de contact (11, 12) avec la suspension de microorganimes (16) sont également disposées parallèlement à l'interface entre la suspension de microorganisme et le milieu de culture. Les deux surfaces de contact (11, 12), en vue perpendiculaire aux plans parallèles formé par les surfaces de contact (11,12) et leurs aires respectives S1 et S2, ne se chevauchent pas et présentent un contour formé d'un diamètre de cercle et de l'arc correspondant. La hauteur h ainsi que les distances h1 et h2 sont également représentées. Dans ce mode de réalisation, le contour des surfaces de contact (11, 12), coopèrent avec la paroi de la boite de Petri afin de répartir la suspension sur deux uniques épaisseurs et d'empêcher toute fuite de la suspension hors des aires des surfaces de contact S₁ et S₂.
La figure 2B représente l'applicateur (10) ayant réparti une suspension (16) sur un milieu de culture déshydraté (19) disposé sur un support (15) sous forme de film. Un film protecteur (20) est disposé sur la suspension de microorganismes (16). Les deux surfaces de contact (11, 12) avec la suspension de microorganismes répartissent la suspension sur deux épaisseurs via le film protecteur (20). Les deux surfaces de contact (11, 12) font face à l'interface entre la suspension de microorganisme et le milieu de culture et sont également disposées parallèlement à ladite interface.

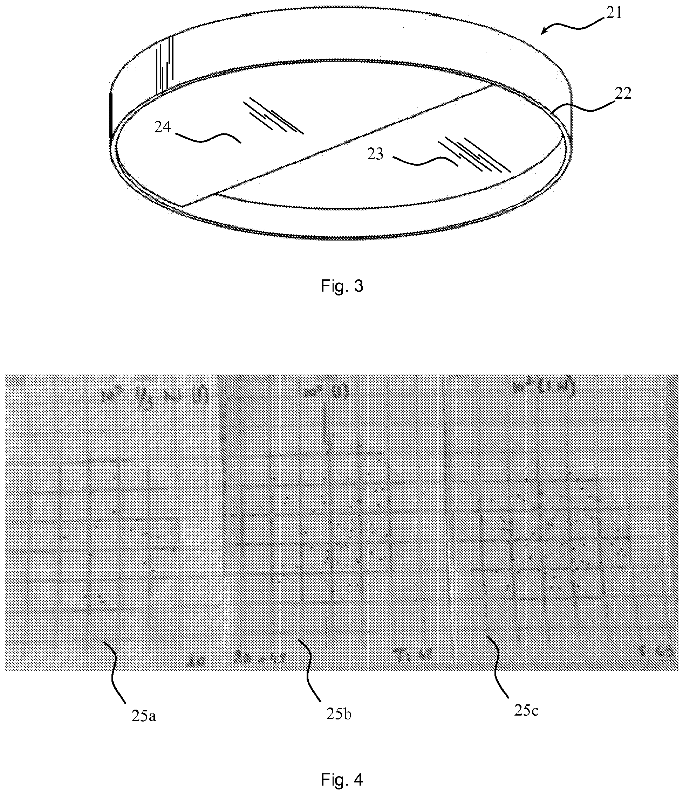

La figure 3 représente un autre mode de réalisation de l'applicateur selon l'invention. Dans ce mode de réalisation, l'applicateur (21) comprend un moyen d'espacement sous la forme d'une bague (22) apte à maintenir les surfaces de contact (23,24) à une distance définie de la partie supérieure du milieu de culture lors de la répartition.
La figure 4 présente trois photographies de milieu de culture Petrifilm® AC 3M Ref. 6400 utilisés avec un applicateur de l'état de l'art (25a, 25c) en comparaison avec un applicateur selon l'invention (25b).

### EXEMPLES

### Méthode

A partir d'une pré-culture sur boite TSA fabriquée par bioMérieux Ref 43011 de la souche *Escherichia coli* ATCC 25922, deux suspensions bactériennes (notées suspension 1 et suspension 2) calibrées à ~0.4 Mac Farland sont préparées de manière à avoir ~10⁸ UFC/mL.
Chaque suspension est ensuite diluée jusqu'à obtenir 10² UFC/mL.
A partir de la suspension 2 à 10² UFC/mL, une dilution supplémentaire est réalisée pour obtenir -60 UFC/mL. De plus, chaque suspension à 10² UFC/mL est diluée au 1/3.

Des films Petrifilm® AC 3M Ref. 6400 sont ensuite ensemencés avec 1 mL des différentes suspensions à tester. Ces Petrifilm présentent un milieu de culture de 6cm de diamètre. Ces milieux de culture sous forme déshydratée sont utilisés pour le dénombrement de la flore totale. Le film protecteur du Petrifilm® est ensuite rabaissé sur l'échantillon.

### Exemple 1 : Dénombrement de la suspension bactérienne 1 à l'aide d'un dispositif selon l'invention

Une série de deux premiers films est ensemencée avec la suspension 1 diluée au 1/3. Une seconde et une troisième séries de deux films sont ensemencées avec la suspension 1 sans dilution.
Un applicateur plastique de l'état de l'art, dénommé applicateur 3M, présentant une surface de contact plane, est placé au centre des films Petrifilm® de la première et troisième série. Un applicateur plastique selon l'invention dénommé applicateur bioMérieux LTN est utilisé. Cet applicateur est circulaire et présente deux surfaces de contacts. La plus petite distance entre les deux surfaces de contacts est de 0,25mm. L'applicateur est placé au centre des films Petrifilm® de la seconde série sur le film protecteur. Une entretoise de 0,25 mm est également disposée en périphérie des surfaces de contact. Les épaisseurs respectives des volumes de suspensions réparties par l'applicateur sont alors de 0.25 mm (D1/2) et 0.50 mm (D1). D1 correspondant à l'épaisseur de l'entretoise additionnée à la plus petite distance entre les deux surfaces de contact. Cet applicateur permet de répartir un échantillon en deux volumes d'épaisseur différentes et donc en deux zones d'épaisseurs différentes qui simulent deux dilutions de l'échantillon à 2/3 et 1/3, chacune des surfaces de contact correspondant à une zone.

Les suspensions sont uniformément réparties en exerçant une légère pression au centre des applicateurs en plastique. L'inoculum est ainsi réparti sur la totalité de la zone de croissance avant que le gel ne se forme.
Les trois films Petrifilm® sont ensuite incubés à 35°C.

La souche d'*E. coli* choisie présentant une bonne croissance, les colonies sont dénombrables dès 24h à 35°C, et une prolongation d'incubation n'est donc pas envisagée.

Les ensemencements réalisés avec l'applicateur 3M montrent après incubation une répartition homogène des colonies sur le film de dénombrement comme décrit dans la documentation 3M.
La figure 4 présente trois photos des trois films ensemencés prisent après 24h d'incubation. Un dénombrement est effectué.
Le premier film (22a), ensemencé avec la suspension 1 diluée au 1/3 et appliquée avec l'applicateur 3M comporte 20 colonies dénombrées.
Le second film (22b), ensemencé avec la suspension 1 sans dilution et appliquée avec l'applicateur bioMérieux LTN comporte 20 colonies dans une première zone et 48 colonies dans une seconde zone soit un total de 68 colonies.
Le troisième film (22c), ensemencé avec la suspension 1 sans dilution et appliquée avec l'applicateur 3M comporte 69 colonies dénombrées.

Les ensemencements réalisés avec l'applicateur bMx LTN montrent après incubation une présence variable des colonies sur le film, en fonction de la zone considérée, comme attendu.

L'applicateur LTN permet le dénombrement du Petrifilm sur la totalité de sa surface ou par zone.
Pour chaque suspension, les résultats obtenus par mL, c'est à dire en utilisation la surface totale des Petrifilm, sont équivalents quel que soit l'applicateur utilisé.
Le dénombrement par applicateur LTN, en zone peu concentrée, correspond au tiers de la concentration globale ou de la concentration par mL. Les résultats correspondent d'une part au tiers du dénombrement total des Petrifilm sur suspensions non diluées, et d'autre part au dénombrement total des suspensions diluées au 1/3, avant ensemencement.
Les résultats du dénombrement sont :

| | Première série, solution 1 à 10² UFC/mL diluée au 1/3 - Applicateur 3M | Seconde série, solution 1 à 10² UFC/mL - Applicateur bioMérieux LTN | Première série, solution 1 à 10² UFC/mL-Applicateur 3M |
|---|---|---|---|
| Nombre de colonies du premier film | 20 | 20+48=68 | 69 |

Dans un mode de réalisation alternatif, une entretoise de 3 mm est disposée en périphérie des surfaces de contact et les épaisseurs respectives des volumes de suspensions réparties par l'applicateur sont alors de 3 mm (D2/2) et 6 mm (D2), le volume de suspension utilisé pour ensemencer le milieu est alors adapté. Dans un autre mode de réalisation alternatif, une entretoise de 3 mm est disposée en périphérie des surfaces de contact et les épaisseurs respectives des volumes de suspensions réparties par l'applicateur sont alors de 3 mm (D3/3) et 9 mm (D3), le volume de suspension utilisé pour ensemencer le milieu est alors adapté.

### Exemple 2 : Dénombrement de la suspension bactérienne 2 à l'aide d'un dispositif selon l'invention

Une série de deux premiers films est ensemencée avec la suspension 2 diluée au 1/3. Une seconde et une troisième séries de deux films sont ensemencées avec la suspension 2 sans dilution
Un applicateur plastique de l'état de l'art, dénommé applicateur 3M, présentant une surface plane, est placé au centre des films Petrifilm® de la première et troisième série. Un applicateur plastique selon l'invention et une entretoise selon l'exemple 1sont placés au centre des films Petrifilm® de la seconde série. Une entretoise de 0,25 mm est disposée en périphérie des surfaces de contact. Les épaisseurs respectives des volumes de suspensions réparties par l'applicateur sont alors de 0.25 mm (D1/2) et 0.50 mm (D1).
Les suspensions sont uniformément réparties en exerçant une légère pression au centre des applicateurs en plastique. L'inoculum est ainsi réparti sur la totalité de la zone de croissance avant que le gel ne se forme.
Les trois films Petrifilm® sont ensuite incubés à 35°C.

La souche d'*E. coli* choisie présentant une bonne croissance, les colonies sont dénombrables dès 24h à 35°C, et une prolongation d'incubation n'est donc pas envisagée. Les ensemencements réalisés avec l'applicateur 3M montrent après incubation une répartition homogène des colonies sur le film de dénombrement comme décrit dans la documentation 3M.

Les ensemencements réalisés avec l'applicateur bioMérieux LTN montrent après incubation une présence variable des colonies sur le film, en fonction de la zone considérée, comme attendu. Dans la seconde série, le premier et le second film présente deux zones de concentration différentes. 64 colonies sont dénombrées dans la première zone du premier milieu, 71 dans la première zone du second milieu. 35 colonies sont dénombrées dans la seconde zone du premier milieu, 34 dans la seconde zone du second milieu.
Les résultats du dénombrement sont :

| | Première série, solution 2 à 10² UFC/mL - Applicateur 3M | Seconde série, solution 2 à 10² UFC/mL - Applicateur bioMérieux LTN | Première série, solution 2 à 10² UFC/mL diluée au 1/3 - Applicateur 3M |
|---|---|---|---|
| Nombre de colonies du premier film | 111 | 64 + 35 = 99 | 35 |
| Nombre de colonies du second film | 101 | 71 + 34 = 105 | 35 |

### Exemple 3 : Dénombrement de la suspension bactérienne 2 à 60 UFC/mL à l'aide dispositif selon l'invention

Un première série de deux films est ensemencée avec la suspension 2 diluée à 60 UFC/mL. Une seconde série deux films est également ensemencée avec la suspension 2 diluée à 60 UFC/mL
Un applicateur 3M est placé au centre des films Petrifilm® de la première série. Un applicateur bioMérieux LTN et une entretoise selon l'exemple 1, sont placés au centre des films Petrifilm® de la seconde série. Les suspensions sont uniformément réparties en exerçant une légère pression au centre des applicateurs en plastique. L'inoculum est ainsi réparti sur la totalité de la zone de croissance avant que le gel ne se forme.

Les quatre films Petrifilm® sont ensuite incubés à 35°C.

La souche d'*E. coli* choisie présentant une bonne croissance, les colonies sont dénombrables dès 24h à 35°C, et une prolongation d'incubation n'est donc pas envisagée.

Les ensemencements réalisés avec l'applicateur 3M montrent après incubation une répartition homogène des colonies sur le film de dénombrement comme décrit dans la documentation 3M.
Un dénombrement est effectué.
La première série de films ensemencée avec la suspension 2 diluée à 60 UFC/mL appliquée avec l'applicateur 3M comporte 67 et 48 colonies dénombrées.
La seconde série de films ensemencée avec la suspension 2 diluée à 60 UFC/mL appliquée avec l'applicateur bioMérieux LTN comporte 36 colonies dans une première zone et 18 colonies dans une seconde zone soit un total de 54 colonies pour le premier film 47 colonies pour le second film.
Les résultats du dénombrement sont :

| | Première série, solution 2 diluée à 60 UFC/ml - Applicateur 3M | Seconde série, solution 2 diluée à 60 UFC/ml - Applicateur bioMérieux LTN |
|---|---|---|
| Nombre de colonies du premier film | 67 | 36 + 18 = 54 |
| Nombre de colonies du second film | 48 | 32 + 15 = 47 |

Ces trois exemples démontrent la reproductibilité d'un dénombrement de bactéries, selon un taux de dilution défini par la forme des surfaces de contacts d'un applicateur disposé sur un milieu de culture non sélectif pour microorganismes.
L'applicateur bioMérieux LTN a conduit à des résultats cohérents et répétables, tout en réduisant le nombre de dilutions et de films à ensemencer.

## Revendications

1. Dispositif comprenant un applicateur apte à répartir une suspension de microorganismes sur un milieu de culture lui-même déposé sur un support, **caractérisé en ce que**, dans sa position de contact avec la suspension de microorganismes, l'applicateur comprend au moins deux surfaces de contact avec la suspension de microorganismes, chaque surface définit un plan, les au moins deux plans étant parallèles l'un par rapport à l'autre et étant distants d'une hauteur h, les au moins deux plans parallèles font face à l'interface entre ladite suspension de microorganismes et le milieu de culture et sont également disposés parallèlement à ladite interface.

2. Dispositif selon la revendication 1 **caractérisé en ce que**, h est égal à h₁-h₂, h₁ étant la distance entre la première surface de contact et l'interface, h₂ étant la distance entre la seconde surface de contact et l'interface et que le ratio h₁/h₂ est supérieur à 1 et inférieur ou égal à 100.

3. Dispositif selon la revendication 2 **caractérisé en ce que** S₁ correspond à l'aire de la première surface de contact de l'applicateur et S₂ correspond à l'aire de la seconde surface de contact de l'applicateur et que le ratio (h₁*S₁)/(h₂*S₂) est supérieur à 1 et inférieur ou égal à 1000.

4. Dispositif selon la revendication 3 **caractérisé en ce qu'**en vue perpendiculaire aux plans parallèles, S₁ et S₂ ne se chevauchent pas de même que les surfaces de contact

5. Dispositif selon l'une quelconque des revendications 2 à 4 **caractérisé en ce que** h₁/h₂ est égal à 3

6. Dispositif selon l'une quelconque des revendications 1 à 5 **caractérisé en ce qu'**il comprend au moins un moyen d'espacement entre les surfaces de contact de l'applicateur et le support ou l'interface.

7. Dispositif selon la revendication 6 **caractérisé en ce que** le moyen d'espacement est solidaire de l'applicateur.

8. Dispositif selon la revendication 6 **caractérisé en ce que** le moyen d'espacement est indépendant de l'applicateur tel qu' au moins une entretoise rapportée.

9. Dispositif selon l'une quelconque des revendications 1 à 8 **caractérisé en ce qu'**il comprend un moyen périphérique de confinement de la suspension de microorganismes.

10. Procédé d'isolement de microorganismes contenus dans une suspension de microorganismes utilisant un dispositif tel que défini à l'une quelconque des revendications 1 à 9 comprenant les étapes suivantes :
a) déposer une suspension de microorganismes sur un milieu de culture lui même déposé sur un support,
b) placer un applicateur sur la suspension, ledit applicateur répartissant la suspension en au moins deux volumes différents et déterminés, et
c) incuber le milieu de culture pendant un temps et à une température prédéterminés permettant la croissance des microorganismes.

11. Procédé selon la revendication 10 comprenant une étape intermédiaire entre les étapes a) et b) consistant à placer un film protecteur sur la suspension de microorganismes, l'étape b) consistant alors à placer un applicateur sur ledit film.

12. Procédé selon l'une quelconque des revendications 10 ou 11 comprenant une étape supplémentaire d) consistant à dénombrer les colonies issues de ces microorganismes.

13. Procédé selon l'une quelconque des revendications 10 à 12 dans lequel le milieu de culture est déshydraté.

14. Procédé selon l'une quelconque des revendications 10 à 13 comprenant une étape préliminaire à l'étape b) consistant à placer une entretoise rapportée sur le milieu de culture

15. Procédé selon l'une quelconque des revendications 10 à 14 comprenant une étape préliminaire à l'étape a) consistant à placer un moyen périphérique de confinement sur le milieu de culture

16. Utilisation d'un dispositif selon l'une des revendications 1 à 9 pour la mise en oeuvre d'un procédé d'isolement de microorganismes.

## Patentansprüche

1. Vorrichtung, umfassend einen Applikator, der zum Verteilen einer Suspension von Mikroorganismen auf einem Kulturmedium ausgelegt ist, das seinerseits auf einen Träger aufgebracht ist, **dadurch gekennzeichnet, dass** der Applikator in seiner Kontaktposition mit der Suspension von Mikroorganismen mindestens zwei Kontaktoberflächen mit der Suspension von Mikroorganismen umfasst, wobei jede Oberfläche eine Ebene definiert, wobei die mindestens zwei Ebenen zueinander parallel und voneinander durch eine Höhe h beabstandet sind, wobei die mindestens zwei parallelen Ebenen der Grenzfläche zwischen der Suspension von Mikroorganismen und dem Kulturmedium gegenüberliegen und außerdem parallel zu der Grenzfläche angeordnet sind.

2. Vorrichtung nach Anspruch 1, **dadurch gekennzeichnet, dass** h gleich h₁-h₂ ist, wobei h₁ der Abstand zwischen der ersten Kontaktoberfläche und der Grenzfläche ist, h₂ der Abstand zwischen der zweiten Kontaktoberfläche und der Grenzfläche ist und das Verhältnis h₁/h₂ größer als 1 und kleiner als oder gleich 100 ist.

3. Vorrichtung nach Anspruch 2, **dadurch gekennzeichnet, dass** S₁ der Fläche der ersten Kontaktoberfläche des Applikators entspricht und S₂ der Fläche der zweiten Kontaktoberfläche des Applikators und dass das Verhältnis (h₁ *S₁)/(h₂ *S₂) größer als 1 und kleiner als oder gleich 1000 ist.

4. Vorrichtung nach Anspruch 3, **dadurch gekennzeichnet, dass**, senkrecht zu den parallelen Ebenen betrachtet, S₁ und S₂ sich nicht überlappen, ebenso wie die Kontaktoberflächen.

5. Vorrichtung nach einem der Ansprüche 2 bis 4, **dadurch gekennzeichnet, dass** h₁/h₂ gleich 3 ist.

6. Vorrichtung nach einem der Ansprüche 1 bis 5, **dadurch gekennzeichnet, dass** sie mindestens ein Beabstandungsmittel zwischen den Kontaktoberflächen des Applikators und dem Träger oder der Grenzfläche umfasst.

7. Vorrichtung nach Anspruch 6, **dadurch gekennzeichnet, dass** das Beabstandungsmittel mit dem Applikator einstückig ist.

8. Vorrichtung nach Anspruch 6, **dadurch gekennzeichnet, dass** das Beabstandungsmittel unabhängig von dem Applikator ist, wie mindestens ein befestigter Abstandshalter.

9. Vorrichtung nach einem der Ansprüche 1 bis 8, **dadurch gekennzeichnet, dass** sie ein peripheres Eingrenzungsmittel für die Suspension von Mikroorganismen umfasst.

10. Verfahren zur Isolation von Mikroorganismen, die in einer Suspension von Mikroorganismen enthalten sind, unter Verwendung einer Vorrichtung nach einem der Ansprüche 1 bis 9, das die folgenden Schritte umfasst:
a) Aufbringen einer Suspension von Mikroorganismen auf ein Kulturmedium, das seinerseits auf einen Träger aufgebracht ist,
b) Platzieren eines Applikators auf der Suspension, wobei der Applikator die Suspension in mindestens zwei unterschiedliche und bestimmte Volumina aufteilt, und
c) Inkubieren des Kulturmediums während einer festgelegten Zeit und bei einer festgelegten Temperatur, die das Wachstum der Mikroorganismen ermöglichen.

11. Verfahren nach Anspruch 10, das einen Zwischenschritt zwischen den Schritten a) und b) umfasst, in dem man einem Schutzfilm auf die Suspension von Mikroorganismen aufbringt, wobei Schritt b) dann aus dem Aufbringen eines Applikators auf den Film besteht.

12. Verfahren nach einem der Ansprüche 10 oder 11, das einen weiteren Schritt d) umfasst, in dem man die aus den Mikroorganismen hervorgegangenen Kolonien zählt.

13. Verfahren nach einem der Ansprüche 10 bis 12, wobei das Kulturmedium dehydratisiert ist.

14. Verfahren nach einem der Ansprüche 10 bis 13, das einen Schritt b) vorausgehenden Schritt umfasst, in dem man einen befestigten Abstandshalter auf das Kulturmedium aufbringt.

15. Verfahren nach einem der Ansprüche 10 bis 14, das einen Schritt a) vorausgehenden Schritt umfasst, in dem man ein peripheres Eingrenzungsmittel auf das Kulturmedium aufbringt.

16. Verwendung einer Vorrichtung nach einem der Ansprüche 1 bis 9 für die Durchführung eines Verfahrens zur Isolation von Mikroorganismen.

## Claims

1. A device comprising an applicator capable of dispensing a suspension of microorganisms on a culture medium which is itself deposited on a support, **characterized in that**, in its position of contact with the suspension of microorganisms, the applicator comprises at least two surfaces of contact with the suspension of microorganisms, each surface defines a plane, the at least two planes being parallel to one another and having a distance between them of a height h, the at least two parallel planes face the interface between said suspension of microorganisms and the culture medium and are also arranged parallel to said interface.

2. The device as claimed in claim 1, **characterized in that** h is equal to h₁-h₂, h₁ being the distance between the first surface of contact and the interface, h₂ being the distance between the second surface of contact and the interface, and that the h₁/h₂ ratio is greater than 1 and less than or equal to 100.

3. The device as claimed in claim 2, **characterized in that** S₁ corresponds to the area of the first surface of contact of the applicator and S₂ corresponds to the area of the second surface of contact of the applicator, and that the (h₁ × S₁)/(h₂ × S₂) ratio is greater than 1 and less than or equal to 1000.

4. The device as claimed in claim 3, **characterized in that**, viewed perpendicular to the parallel planes, S₁ and S₂ do not overlap, likewise the surfaces of contact.

5. The device as claimed in any one of claims 2 to 4, **characterized in that** h₁/h₂ is equal to 3.

6. The device as claimed in any one of claims 1 to 5, **characterized in that** it comprises at least one means for leaving a space between the surfaces of contact of the applicator and the support or the interface.

7. The device as claimed in claim 6, **characterized in that** the means for leaving a space and the applicator are interdependent.

8. The device as claimed in claim 6, **characterized in that** the means for leaving a space is independent of the applicator, such as an added spacer piece.

9. The device as claimed in any one of claims 1 to 8, **characterized in that** it comprises a peripheral means for confining the suspension of microorganisms.

10. A method for isolating microorganisms contained in a suspension of microorganisms, using a device as defined in any one of claims 1 to 9, comprising the following steps:
a) depositing a suspension of microorganisms on a culture medium which is itself deposited on a support,
b) placing an applicator on the suspension, said applicator dispensing the suspension in at least two different and determined volumes, and
c) incubating the culture medium for a time and at a predetermine temperature allowing the growth of the microorganisms.

11. The method as claimed in claim 10, comprising an intermediate step, between steps a) and b), consisting in placing a protective film on the suspension of microorganisms, step b) then consisting in placing an applicator on said film.

12. The method as claimed in either one of claims 10 and 11, comprising an additional step d) consisting in counting the colonies resulting from these microorganisms.

13. The method as claimed in any one of claims 10 to 12, wherein the culture medium is dehydrated.

14. The method as claimed in any one of claims 10 to 13, comprising a step, preliminary to step b), consisting in placing an added spacer piece on the culture medium.

15. The method as claimed in any one of claims 10 to 14, comprising a step, preliminary to step a), consisting in placing a peripheral confining means on the culture medium.

16. The use of a device as claimed in one of claims 1 to 9 for carrying out a method for isolating microorganisms.
